# EUROPEAN PATENT APPLICATION

(11) **EP 1 469 012 A1**
(43) Date of publication of application: **20.10.2004**
(21) Application number: 03076141.5
(22) Date of filing: 17.04.2003
(51) Int. Cl.: C08F 4/04, C07D 233/24, B65D 77/02

(54) **Polymerisation initiator system**

(71) Applicant: TEMSA International Inc., Danbury, Connecticut 06810 (US)
(72) Inventor: Sarkar, Asim Kumar, Danbury, CT 06810 (US)
(74) Representative: Prins, Adrianus Willem, Mr. Ir.

(57) **Abstract**

The present invention relates to a polymerisation initiator system, in particular a system for aqueous emulsion or solution polymerisation with a polymerisation initiator system comprising an azo-compound as an initiator. The present invention further relates to a method for preparing a polymer, in particular an emulsion or solution polymerisation method, which method comprises the addition of a specific polymerisation initiator system.

## Description

The invention relates to a polymerisation initiator system and to a method of preparing a polymer.

Recently, a number of relatively new and very effective water soluble organic initiators for the polymerisation of monomers in a largely aqueous medium have been made available to the producers of polymers. These initiators, such as water soluble azo-initiators, are usually expensive.

The initiators are usually available as crystalline water-soluble powders. Aqueous solutions of the initiators are not as stable as the dry powder and stock solutions held for any length of time have to be stored under cold conditions. As an alternative it has been proposed to add the initiator to the polymerisation system as a dry powder.

The dry powders, as such, are toxic and irritant to the skin, eyes and/or the lungs. Adequate care has always to be taken to observe stringent safety precautions during all stages of handling the product, during transport to the warehouse and to the plant, such that at no stage personnel is exposed to dust that is inherently formed from such crystalline initiators. Besides the health hazards, there is of course a cumulative loss of expensive material due to the handling of the powder at different stages of transfer of the initiator.

As an alternative, it has been proposed to apply the initiator in the form of granules. However, according to EP-A-0 668 098 there is a large risk and a fear of deterioration of quality during granulation by means of a normal granulation method, because of the deleterious decomposition properties of many initiators, such as azo-compounds. Further, normal granulation methods lead to granules wherein formation of dust cannot be sufficiently eliminated.

In order to address these problems, EP-A-0 668 098 proposes a special process, requiring dedicated equipment for the production of granules of a specific average particle size of 0.01-30 mm, preferably 1.8-5 mm. This approach leaves a number of aspects in the production of water soluble low volume specialty initiators with inherent problems related to safety of multiple handling from the production of the initiator until its actual transfer to the reactor wherein the polymer is produced. Further this approach does not actually address the economics of production of the final form of the product, as the need for dedicated equipment and special process conditions, has a cost-increasing effect on the initiator.

There remains a need for providing alternatives to known initiator delivery systems and/or methods of polymerisation comprising the addition of an initiator delivery system. It is an object of the present invention to provide such an alternative.

It is in particular an object of the invention to address one or more of the above identified problems.

More in particular, the present invention aims to provide a relatively simple and safe method and/or initiator delivery system to enable the producer of the initiator to transfer the initiator from its actual unit of production to the end-user, without undue human exposure of the initiator at various stages of transport until transfer into the polymerisation reaction system.

It has been found that it is possible to suitably add an initiator held in a specific container to a polymerisation system, such as a largely aqueous polymerisation system, to start a polymerisation reaction.

Accordingly, the present invention relates to a polymerisation initiator system, comprising an initiator - in particular an azo-compound - in a container, which container and initiator are both soluble in a polymerisation system, such as a liquid mixture comprising the compound or compounds to be polymerised and optionally other components used in the polymerisation such as a catalyst, an emulsifier and the like.

More in particular the present invention relates to a polymerisation initiator system, comprising a water-soluble container and a water-soluble azo-initiator contained in the container.

The container and the initiator are chosen such that they dissolve in the polymerisation system. It has been found that the polymerisation can suitably be carried out in the presence of the material of which the container is made.

The invention provides a convenient system for delivering a water-soluble organic polymerisation initiator and/or catalyst to a variety of polymerisation processes, in which monomers of various types may be used to produce a usually water-soluble polymer, preferably by aqueous emulsion polymerisation or aqueous solution polymerisation.

The invention is suitable to provide a range of polymers for use in a large number of diverse industries, including consumer industries, such as processing of paper, textile, adhesives, water treatment, food products etc.

A system according to the invention has been found very suitable as a substitute for known polymerisation initiator systems for delivering an initiator in a method wherein the initiator is directly added to the polymerisation system in a measured amount, such as a polymerisation method for the production of water soluble or emulsifyable polymers, in particular such a method wherein it is desired to have an extremely low amount of residual monomer in the final polymer product.

The system is particularly suitable for use in an aqueous emulsion polymerisation. Herein, it has been found that the system dissolves sufficiently fast and the polymerisation is adequately initiated by the initiator. It has further been found of a system of the invention that the material of which the container is made mainly remains in the aqueous bulk phase. It has been found that typically only trace amounts, if noticeable at all, of the material of which the container is made, migrate into the monomer phase (inside the micelles wherein polymerisation takes place) and end up in the final product. Thus, it has been found possible to prepare a polymer without unacceptable levels of the water-soluble material of which the container is made.

It has been found that a system according to the present invention can be introduced conveniently into a polymerisation system by a regular chemical plant operator without any specific training, with a low risk of exposure to the (hazardous) initiator, during application to the polymerisation system.

Dust formation can be avoided essentially completely or at least to a large extent in comparison to application of the initiator without the container.

The initiator can conveniently be applied very accurately without substantial loss of initiator due to dust formation or spoiling during application to the polymerisation system.

The term water-soluble as defined herein is a substance (such as the azo-initiator or the container) which is soluble in the polymerisation system under reaction conditions existing in the system for which the polymerisation initiation system is intended, in particular in an aqueous polymerisation system. Preferably the water-solubility of a substance is at least 0.1 g/100 g water at 40 °C, more preferably at least 1 g/100 g water at 40 °C, even more preferably at least 4 g/100 g water at 40 °C.

The container may have any form. It may for example have the form of a box, tube or capsule. Preferably the container is a bag or the like.

Preferably, the container is made of a water-soluble polymer. The polymer is preferably chosen such that it has a low interference with or detrimental effect to the polymeric material that is prepared by polymerisation after initiation by the polymerisation initiation system.

Preferred examples of suitable water-soluble polymers include polyvinyl alcohols (PVAs) and cellulosic polymers, in particular water-soluble cellulose derivatives. The skilled person will know how to choose suitable derivatives, based upon common knowledge and the information disclosed in the present description and claims.

Particularly preferred is a container comprising a polyvinylalcohol as the major component of which the container is made or essentially consisting of a polyvinylalcohol.

The container, such as the bag, may have been made in any way. Very good results have been achieved with an extruded container, such as an extrusion blown bag, and with a cast container, such as a bag made from a cast film.

Suitable containers may be made in any way and are commercially available e.g. from Aquafilm Ltd, Hartlesbury, UK (such as L330™) or Innopac, Winston-Salem NC, USA (such as EF-220™).

The size of the container may suitably be chosen based upon the amount of initiator it needs to hold, common general knowledge and the information disclosed in the present description and claims.

The initiator is a compound that is capable of initiating a polymerisation reaction, in particular a compound that forms at least one radical under reaction conditions and thus can start a polymerisation such as the polymerisation of a monomer comprising at least one vinyl group.

The azo-initiator is an azo-compound comprising a -N=N- moiety. Preferably the compound is an organic compound.

The compound typically decomposes after dissolution in a polymerisation system - e.g. by adding heat - usually under formation of nitrogen and two free radicals. The free radicals are capable of acting as an initiator for a radical polymerisation such as a vinyl polymerisation.

Preferably the azo-compound is represented by the formula I

((R¹R²YC)-N=N-(CZR³R⁴)].(2/n)HXⁿ⁻ Formula I

wherein
R¹, R², R³ and R⁴ each represent the same or a different optionally substituted alkyl or cycloalkyl group, preferably a C₁-C₆ alkyl or C₃-C₆ cycloalkyl, more preferably methyl or ethyl;
Y and Z each represent the same or a different group represented by the formula 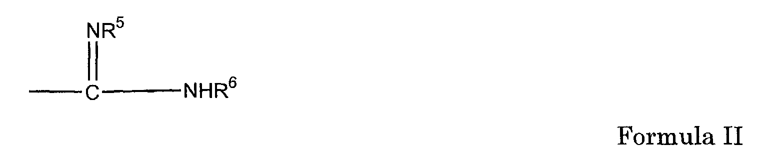 or 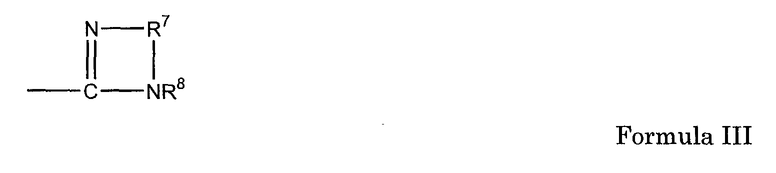 wherein R⁵ is a hydrogen atom or an optionally substituted alkyl, allyl or phenyl group, wherein in case of an alkyl or allyl the alkyl respectively allyl preferably is C₁-C₆, and wherein the case of a phenyl, the phenyl group is preferably tolyl, xylyl or cumyl
wherein R⁶ represents hydrogen or an optionally substituted alkyl or cycloalkyl group, preferably a hydrogen or C₁-C₆ alkyl or C₃-C₆ cycloalkyl
wherein R⁷ is an optionally substituted alkylene group, preferably a C₁-C₆ alkylene, more preferably having one to three carbon atoms and even more preferably having one to three hydroxyl groups
wherein R⁸ is a hydrogen atom or a hydroxyalkyl group, preferably a hydrogen atom or a C₁-C₆ hydroxyalkyl group
and X is an anion, n representing the valence, preferably a monovalent anion (n=1), more preferably chloride, bromide or acetate.

Very good results have been achieved with a compound according to formula I wherein Y and Z each are represented by formula II, in particular
wherein R¹ to R⁴ are each independently methyl or ethyl and wherein R⁵ and R⁶ are hydrogen or methyl.

Of these compounds, a salt of 2,2'-Azobis(2-amidinopropane), especially a dihydrochloride salt (formula IV), is particularly preferred.

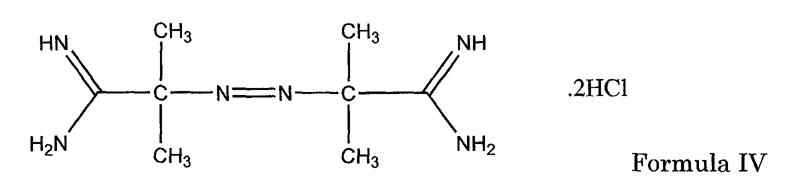

Very good results have been achieved with a compound according to formula I wherein Y and Z each are represented by formula III, wherein R¹ to R⁴ are each independently methyl or ethyl, wherein R⁷ is an ethylene group and R⁸ preferably is a hydrogen atom or a methyl.

Of these compounds, a salt of 2,2'-azobis[2-(2-imidazolin-2-yl)-propane], especially a dihydrochloride salt (Formula V), is particularly preferred.

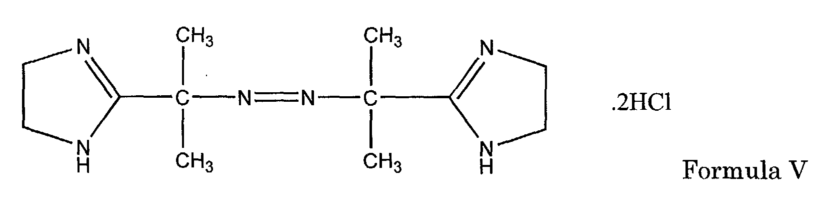

Other suitable azo-initiators include azo-compounds represented by the formula

[O-(C=O)-A-(CR⁹(CN))-N=N-(CR¹⁰(CN))-B-(C=O)-O]²⁻.(2/n)Yⁿ Formula VI

wherein A and B represent the same or a different unsubstituted or substituted alkylene, preferably having one to six carbon atoms and

R⁹ and R¹⁰ each represent the same or a different optionally substituted alkyl group, preferably having one to six carbon atoms.

Y is a mono or bivalent cation and represents the valence of Y; wherein Y is preferably an alkali metal - in particular sodium or potassium - an alkaline earth metal - in particular magnesium, calcium or barium - or ammonium and more preferably sodium or potassium.

For practical reasons the azo-initiator, for instance as shown in formula I or VI, is preferably symmetrical, i.e. the groups on either side of the -N=N- are preferably identical (in said formula's: R¹ = R³, R² = R⁴, Y=Z, respectively R⁹ = R¹⁰ and A=B).

The substituents of the optionally substituted hydrocarbon groups in the above formulae may be selected from the group consisting of halogen atoms, hydroxyl, and the like. Preferably at least the majority of the substituents, if present, are hydroxyl.

Very good results have been achieved with an initiator wherein essentially all of the optionally substituted hydrocarbon groups are unsubstituted.

The initiator in the container such as the bag preferably has a particulate form. It may have the form of a powder, crystals, granules or it may be a mixture of any of these. The particles may be relatively small, e.g. having a number average particle size of up to about 1.5 mm, more in particular of up to about 1 mm - or relatively large, e.g having a number average particle size of more than 1 mm, more in particular of more than about 1.5 mm up to 5 mm or more. The particles of the initiator may have any shape, e.g. as described in EP-A-6 689 098 or the prior art described therein. No special attention is required to ensure that the particles are highly spherical to avoid dust exposure to the environment. In this respect, it is a further advantage of the invention that the geometric properties and resistance to break-down of the form in which the initiator is originally present in the container, are not particularly important to successfully avoid exposure to dust.

For practical reasons, the initiator is preferably provided into the container in the form the initiator has after having been manufactured (and dried), typically having the form of a crystalline material, comprising microcrystal particles and macro-crystal particles.

The container comprising the initiator may be sealed in any way, a seal, a lid or by heat-sealing. Very suitable is *inter alia* a wrapping with a water-soluble seal - such as water-soluble tape or tie-wrap - a seal formed by gluing with water-soluble glue or a seal formed by heat-sealing. Very good results have been achieved with a water-soluble wrapping, such as tape. This has been found to be any easy and satisfactory way of sealing the container, in particular a bag. Further, the wrapping may be applied without any further treatment giving rise to physico-chemical changes in the material, such as an undesirable formation of insoluble particles in the wrapping material or the container material. Very suitable is a seal made of a water-soluble polyvinylalcohol.

The container with initiator may be packed alone or together with one or more other containers in an outer container, such as a polymer bag, e.g. a polyethylene bag or the like.

Preferably, the outer container is provided with a label specifying the specific amount of initiator it contains and more preferably with a certificate of analysis.

The amount of initiator in the container may be varied within wide limits. For ease of handling, it is preferred that the container holds 1 g to 25 kg, preferably 100 g to 10 kg, more preferably 500 g to 5 kg of initiator. Preferably the initiator is present in a measured amount, indicating a tolerance. The tolerance, preferably is as specified by the end-user and will depend upon the polymerisation system

Generally, the initiator is the major component held in the container. Preferably the initiator provides about 75 wt. % to 100 wt. % of the contents of the container, more preferably 90-99.9 wt. % of the contents of the container, even more preferably about 95-99 wt. % of the contents of the container.

Optionally, the system further comprises one or more additives, which are typically water-soluble. Preferred examples of additives include anti-foaming agents and diluent materials.

The antifoam agent may be present to suppress foaming during dissolution of the polymerisation initiator system. Suitable antifoam agents are known in the art and include for example Foam-Stopper™ (Harlow chemicals).

The polymerisation initiator system may comprise a diluent material. This is typically a cheap material that does not adversely affect the polymerisation to an unacceptable level. It may for instance be a material that has been used in the manufacture of the initiator, such as ammonium chloride.

The present invention further relates to a method for preparing a polymer, wherein an initiator in a container is added to a polymerisation system - preferably a liquid such as an emulsion - and the initiator and container are dissolved in the polymerisation system.

A method for preparing a polymer in accordance with the invention typically comprises
- adding a container - which is soluble in a liquid wherein the polymerisation is to be carried out - said container comprising an initiator, in particular an azo-initiator;
- dissolving the container and the initiator;
- mixing the liquid with at least one compound to be polymerised (such as a monomer) and optionally other components for carrying out the polymerisation; and
- letting the monomer polymerise.

In principle the addition of the container can be done before, during or after adding any of the other components for polymerisation to the liquid. It is however preferred to first add at least the monomer(s) to the liquid and, when used, the emulsifier(s) before adding the container. More preferably the container is added after substantially all other components have been added to the liquid.

A method according to the present invention is very suitable for solution or for emulsion polymerisation.

Very good results have been achieved with a method wherein the liquid comprises water or an aqueous medium, in particular in an emulsion polymerisation as the phase wherein the emulsifier(s) and monomer(s) are dispersed.

In principle, a method according to the invention can be any type of polymerisation that the initiator can initiate, in particular any radical polymerisation reaction, such as a radical polymerisation in an aqueous emulsion.

Suitable reaction conditions are generally known in the art and have been amply illustrated in e.g. commercial brochures on the initiators, scientific publications and patent literature.

The present invention has been found very suitable to polymerise at least one monomer selected from the group consisting of acrylic acid, acryl amide, acrylesters (e.g. ethyl acrylate), vinyl acetate, acetonitrile and styrene. Very good results have been achieved with the polymerisation of acrylic acid and/or acryl amide.

It has further been found that the present invention is very suitable for use in an application selected from the group consisting of the aqueous polymerisation of acrylamide for use as a flocculants, the copolymerisation of acrylamide with acrylic acid, the manufacture of cationic polymers, the manufacture of polymer emulsions and the production of solid anionic acrylic polymers.

The present invention further relates to the use of a polymerisation initiator system as an initiator for a polymerisation reaction, in particular an emulsion polymerisation reaction.

The present invention further relates to the use of a polymerisation initiator system for reducing dust formation in the application of an initiator to a polymerisation system.

The present invention further relates to the use of a polymerisation initiator system for reducing a health hazard risk.

The present invention further relates to a method for preparing a polymerisation initiator system as defined in the present description or claims, wherein the initiator and optionally additives are introduced into the container, after which the container is sealed.

The amount of initiator and optionally of additives is preferably as specified by the customer (such as the producer of the polymer to be prepared with the aid of the system). Thus in this aspect of the invention, a custom-made initiator delivery system is provided.

The present invention further relates to a method of handling an a polymerisation initiator system as described in the present description or claims, wherein the system is transferred from a polymerisation initiator system manufacturing site to a polymer production site and integrally introduced into a polymerisation reactor. The system preferably remains closed throughout the whole transfer.

The invention will now be elucidated by the following examples

### EXAMPLE 1

A 12"x 10" PVA bag (L330^{TM} Aquafilm Ltd.) was filled with 2,2'-Azobis(2-amidinopropane) dihydrochloride (V-50^{TM} Wako Chemicals, GmbH, Neuss, Germany) and sealed by wrapping water-soluble polyvinyl alcohol tape around the opening. The weight of the PVA bag (i.e. the PVA content) was approximately 0.4 wt. % based upon the weight of the initiator.

A polymerisation mixture was prepared by adding a acrylamide/acrylic acid mixture (90 : 10 mole to mole) to water (in the absence of emulsifyer) to a final concentration of 5 wt. % monomer per kg water and adding the bag with initiator. The amount of water in the mixture was sufficient to obtain a 0.05 wt. % initiator concentration after dissolution of the bag and initiator. In the final acrylamide/acrylic acid polymer, the PVA content was found to be only a few ppm.

## Claims

1. Polymerisation initiator system, comprising a water-soluble container and a water-soluble azo-initiator inside the container.

2. Polymerisation initiator package according to claim 1, wherein the water-soluble container is a bag.

3. Polymerisation initiator system according to claim 1 or 2, wherein the container is made of a water-soluble polymer.

4. Polymerisation initiator system according to claim 3, wherein the water-soluble polymer is a water-soluble cellulosic polymer or polyvinylalcohol.

5. Polymerisation initiator system according to claim 3 or 4, wherein the container is an extruded container.

6. Polymerisation initiator system according to any one of the preceding claims, wherein the azo-initiator is selected from the group consisting of compounds represented by the formula
(R¹R²YC)-N=N-(CZR³R⁴).(2/n)HXⁿ⁻ Formula I
wherein
R¹, R², R³ and R⁴ each represent the same or a different alkyl group or cycloalkyl group
Y and Z each represent the same or a different group represented by the formula 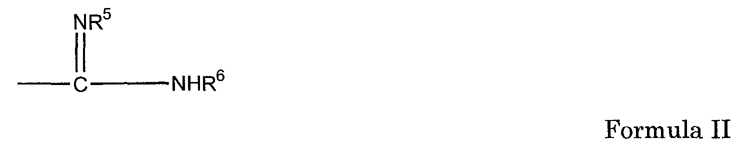 or 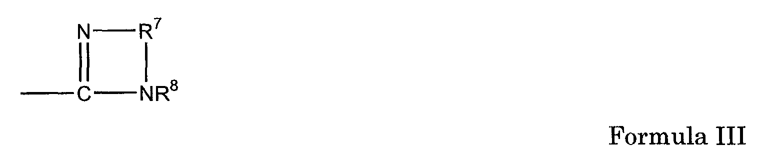 R⁵ is a hydrogen atom or an optionally substituted alkyl, allyl or phenyl group
R⁶ is a hydrogen atom or an optionally substituted alkyl or phenyl group
R⁷ is an optionally substituted alkylene group
R⁸ is a hydrogen atom or a hydroxyalkyl group
X is a anion, wherein n represents its valence, and X is preferably a monovalent anion, more preferably chloride, bromide or acetate.

7. Polymerisation initiator system according to claim 6, wherein the the azo-initiator is 2,2'-Azobis(2-amidinopropane), 2,2'-azobis[2-(2-imidazolin-2-yl)propane] or a salt thereof

8. Polymerisation initiator system according to any one of the preceding claims, wherein the amount of azo-initiator in the container is in the range of 1 g to 25 kg, preferably of 100 to 10 kg.

9. Polymerisation initiator system according to any one of the preceding claims, wherein the container comprises at least one component selected from the group consisting of anti-foaming agents and diluent materials.

10. Method of polymerisation, comprising adding a container comprising an initiator to a polymerisation system and dissolving the initiator and container in the polymerisation system.

11. Method according to claim 10, wherein the polymerisation system is an emulsion polymerisation system or a solution polymerisation system.

12. Method according to claim 10 or 11, wherein the polymerisation system is an aqueous polymerisation system.

13. Method according to any one of the claims 10-12, wherein the polymerisation system comprises at least one monomer selected from the group consisting of acrylic acid, acryl amide, acrylesters (e.g. ethyl acrylate), vinyl acetate, acetonitrile and styrene, preferably from the group consisting of acrylic acid and acryl amide.

14. Method according to any one of the claims 10-13, wherein the container and initiator are added in the form of a polymerisation initiator system as defined in any one of the claims 1-9.

15. Method for preparing a polymerisation initiator system according to any one of the claim 1-9, wherein the water-soluble azo-initiator is introduced into the water-soluble container, after which the container is sealed.

16. Method for handling a polymerisation initiator system according to any one of the claims 1-9, wherein the system is transferred from a polymerisation initiator system manufacturing site to a polymer production site and integrally introduced into a polymerisation reactor.
